(19) Europäisches Patentamt

European Patent Office

Office européen des brevets

(11) Veröffentlichungsnummer: **0 314 902 B1**

# (12) EUROPÄISCHE PATENTSCHRIFT

(45) Veröffentlichungstag der Patentschrift: **02.12.92**

(21) Anmeldenummer: **88114764.9**

(22) Anmeldetag: **09.09.88**

(51) Int. Cl.⁵: **A61B  17/56**

(54) **Einschlaginstrument für chirurgische Klingen.**

(30) Priorität: **02.11.87 DE 3737163**

(43) Veröffentlichungstag der Anmeldung:
**10.05.89 Patentblatt  89/19**

(45) Bekanntmachung des Hinweises auf die
Patenterteilung:
**02.12.92 Patentblatt  92/49**

(84) Benannte Vertragsstaaten:
**DE ES FR GB IT**

(56) Entgegenhaltungen:
**EP-A- 0 241 792**
**FR-A- 1 354 525**
**GB-A- 2 118 474**
**US-A- 3 866 458**

(73) Patentinhaber: **Waldemar Link GmbH & Co**
**Barkhausenweg 10**
**W-2000 Hamburg 63(DE)**

(72) Erfinder: **Keller, Arnold**
**An der Naherfurth 5**
**W-2061 Kayhude(DE)**

(74) Vertreter: **Glawe, Delfs, Moll & Partner Patent-**
**anwälte**
**Postfach 26 01 62 Liebherrstrasse 20**
**W-8000 München 26(DE)**

EP 0 314 902 B1

## Beschreibung

Die Erfindung betrifft ein Einschlaginstrument für chirurgische Klingen, insbesondere Klingenplatten für Keilosteosynthese bei supratuberculärer Keilosteotomie.

Um eine Klinge der im DE-U-81 24 272 angegebenen Art während des Einschlagens sicher fassen und führen zu können, ist ein Instrument bekannt, das einen Schaft mit einem Schlagende und einem Befestigungsende umfaßt, das einen Klemm-Mechanismus zum Befestigen der Klinge aufweist. Dieser umfaßt zwei Klauen, die seitlich über die Längskanten der Klinge fassen und mittels einer Schraube zusammengepreßt werden können, deren Betätigungsglied seitlich von dem Klemm-Mechanismus vorragt. Dies hat den Nachteil, daß das Betätigungsglied in unmittelbarer Nähe des Operationsfeldes gedreht werden muß, um das Einschlaginstrument von der Klinge zu lösen. Denselben Nachteil hat das Werkzeug nach der GB-A 2 118 474, das zum Einschlagen von Knochenklammern eine Spannzange aufweist, deren Backen über Konusflächen von einer in Längsrichtung des Werkzeugs verschraubbaren Hülse zusammenpreßbar sind. Da diese Hülse auf die am Ende des Werkzeugs befindlichen Spannbacken einwirken muß, ist auch sie selbst am Werkzeugende nahe dem Operationsfeld angeordnet.

Der Erfindung liegt die Aufgabe zugrunde, ein Einschlaginstrument dieser Art zu schaffen, das diesen Nachteil nicht aufweist.

Die erfindungsgemäße Lösung besteht darin, das das Betätigungsglied am Ende einer längs des Schafts geführten Stange nahe dem Schlagende angeordnet ist. Vorzugsweise ist es vertieft in einer das Werkzeug führenden Bohrung in dem Schlagende angeordnet. Die Manipulation zum Lösen der Klinge von dem Instrument spielt sich daher an bequemer Stelle und in bequemer Handhabungsrichtung fern vom Operationsfeld ab.

Der Klemm-Mechanismus weist zweckmäßigerweise einen mit dem Schaft fest verbundenen Stempel zur Übertragung der Schlagkraft auf die Klinge sowie eine mittels einer Schraube gegen den Stempel spannbare Klemme auf, so daß ein Teil der Klinge zwischen der Stempelstirnfläche und der Klemme gespannt werden kann. Die Schraube ist dabei mit dem Betätigungsglied verbunden. Die Klemme wird nach einem weiteren Merkmal der Erfindung von zwei aufeinander zu gerichteten Klemmvorsprüngen gebildet, die am Ende von zwei seitlich des Stempels liegenden und verschiebbaren Seitenwänden vorspringen. Die Klemmvorsprünge, die Seitenwände und die Stirnfläche des Stempels schließen zur Aufnahme einer Kopfplatte der Klinge eine Kammer ein, die zumindest an einer Seite offen ist zum Einführen bzw.

Entnehmen der Kopfplatte.

Die Erfindung wird im folgenden näher unter Bezugnahme auf die Zeichnung erläutert, die ein vorteilhaftes Ausführungsbeispiel veranschaulicht. Es zeigen:

Fig. 1     eine teilweise geschnittene Draufsicht,

Fig. 2     eine Seitenansicht und

Fig. 3     einen Schnitt durch das Befestigungsende des Instruments.

Der Schaft 1 des Instruments trägt an seinem Schlagende einen verdickten Aufschlagteil 3 und an seinem Befestigungsende einen Stempel 5 mit einer Stirnfläche 7. Zu einer Seite hin ist die Stirnfläche 7 durch einen Kragen 9 begrenzt.

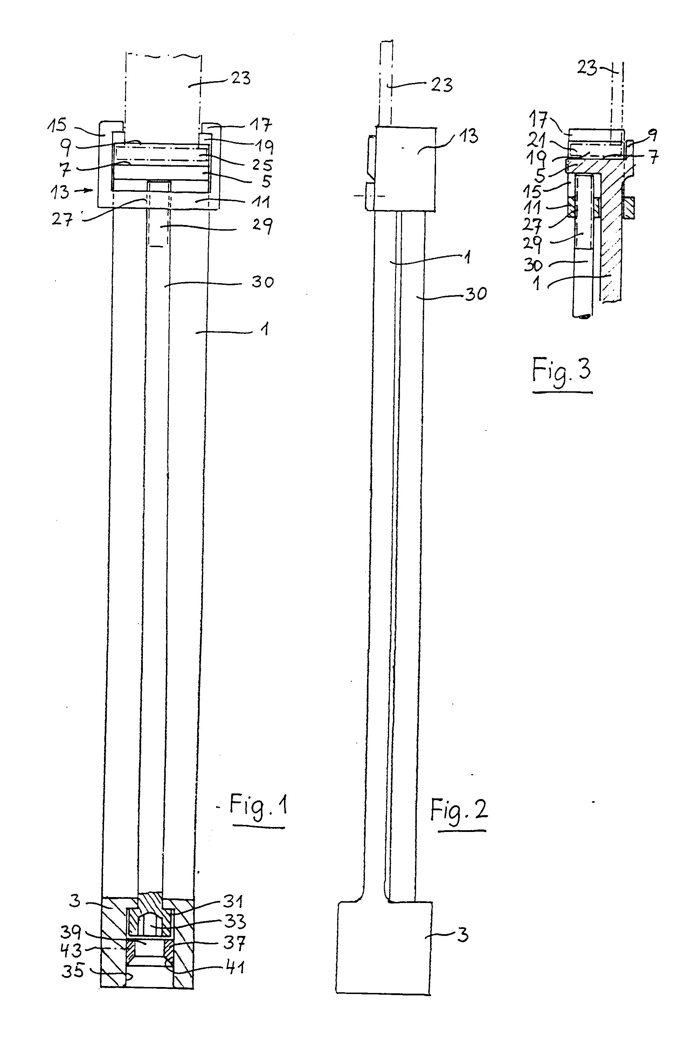

An dem Stempel 1 ist die Platte 11 einer Klaue 13 in Längsrichtung des Stempels geführt, von der aus Seitenwände 15 sich parallel zur Schaftrichtung auf zwei gegenüberliegenden Seiten des Stempels 5 erstrecken, die an ihren Enden Klemmvorsprünge 17 tragen, die parallel zur Stirnfläche 7 des Stempels 5 aufeinander zu ragen. Die Seitenwände 15 und Vorsprünge 17 der Klaue 13 schließen gemeinsam mit der Stirnfläche 7 und dem Kragen 9 des Stempels 5 eine Kammer 19 für die Aufnahme der Kopfplatte 21 einer Klingenplatte 23 ein. Zum Einfügen und Herausnehmen der Kopfplatte ist die Kammer 19 auf der in Fig.3 links erscheinenden Seite offen. Wenn die Kopfplatte 21 in die Kammer 19 eingesetzt ist, greifen die Klemmvorsprünge 17 über die seitlich vorragenden Ränder 25 der Kopfplatte 21.

Die Platte 11 der Klaue 13 enthält parallel zum Schaft 1 eine Gewindebohrung 27, in welche das mit einem entsprechenden Gewinde 29 versehene Ende einer Stange 30 eingreift, die parallel zum Schaft 1 geführt ist und deren anderes als Betätigungsglied 31 mit einem Innensechskant 33 ausgebildetes Ende in einer Bohrung 35 des Aufschlagteils 3 drehbar ist. Sie ist darin durch einen Haltering 37 gehalten, dessen mit dem Innensechskant 33 fluchtende Bohrung 39 einen Durchmesser besitzt, der nicht wesentlich größer ist als der Durchmesser des zugehörigen Innensechskantschlüssels und der eine Einführungsschräge 41 zur Führung dieses Schlüssels aufweist, so daß er leicht und blind eingeführt werden kann.

Durch Drehung der Stange 30 kann die Klaue 13 mit den Klemmvorsprüngen 17 gegen die Stirnfläche 7 des Stempels 5 bzw. von dieser weg bewegt werden.

Soll eine Klingenplatte 23 eingeschlagen werden, wird die Kopfplatte 21 in die Kammer 19 eingeführt und darin zwischen der Stirnfläche 7 des Stempels 5 und den Klemmvorsprüngen 17 durch Drehung der Stange 30 festgeklemmt, wobei der Klingenteil zwischen den Klemmvorsprüngen 17 aus der in Einschlagrichtung weisenden Öffnung der Klaue 13 herausragt.

Nach dem Einschlagen der Klinge kann sie durch entsprechende Drehung der Stange 30 wieder von dem Instrument gelöst werden, wobei das Werkzeug zur Betätigung der Stange in bequemer Haltung und entfernt vom Operationsfeld gehandhabt werden kann.

## Patentansprüche

1. Einschlaginstrument für chirurgische Klingen, insbesondere Klingenplatten für Keilosteosynthese bei supratuberculärer Keilosteotomie, das einen Schaft mit einem Schlagende und einem Befestigungsende umfaßt, das einen Klemm-Mechanismus zum Befestigen der Klinge aufweist, der ein mittels eines Werkzeugs zu drehendes Betätigungsglied besitzt, dadurch gekennzeichnet, daß das Betätigungsglied (31) am Ende einer längs des Schafts (1) geführten Stange (30) nahe dem Schlagende (3) angeordnet ist.

2. Instrument nach Anspruch 1, dadurch gekennzeichnet, daß das Betätigungsglied (31) vertieft in einer das Werkzeug führenden Bohrung (35) in dem Schlagende angeordnet ist.

3. Instrument nach Anspruch 1 oder 2, dadurch gekennzeichnet, daß der Klemm-Mechanismus einen mit dem Schaft (1) fest verbundenen Stempel (5) und eine mittels einer mit dem Betätigungsglied (31) verbundenen Schraube (29) unter Einschluß eines Teils (21) der Klinge (23) gegen den Stempel (5) spannbare Klemme (15,17) umfaßt.

4. Instrument nach Anspruch 3, dadurch gekennzeichnet, daß die Klemme (15,17) zwei seitlich des Stempels liegende Seitenwände (15) und an deren Enden angeordnete, aufeinander zu gerichtete Klemmvorsprünge (17) umfaßt, die zusammen mit der Stirnfläche (7) des Stempels (5) eine quer zur Schaftrichtung offene Kammer (19) zur Aufnahme einer Kopfplatte (21) der Klinge einschließen.

## Claims

1. Driving instrument for surgical blades, particularly for plate blades for cuneiform osteosynthesis in supratubercular cuneiform osteotomy, comprising a shaft with a driving end and a fixing end with a clamping mechanism for fixing the blade, this mechanism having a control element which is rotated with a tool, characterised in that the control element (31) is located at the end of a rod (30) guided along the length of the shaft (1) close to the driving end (3).

2. Instrument in accordance with Claim 1, characterised in that the control element (31) is located recessed in a bore (35) in the driving end, which guides the tool.

3. Instrument in accordance with Claim 1 or 2, characterised in that the clamping mechanism comprises a punch (5), permanently attached to the shaft (1), and a clamp (15, 17) which can be tightened against the punch (5) via a screw (29) connected to the control element (31), such that it includes a section (21) of the blade (23).

4. Instrument in accordance with Claim 3, characterised in that the clamp (15, 17) comprises two side walls (15) situated at the side of the punch and, located on the ends of these side walls, clamp projections (17) aligned towards one another, which, together with the face (7) of the punch (5) enclose an open chamber (19), transverse to the direction of the shaft, to take up a top plate (21) of the blade.

## Revendications

1. Instrument d'insertion par percussion de lames chirurgicales, en particulier de plaques en lame pour ostéosynthèse sphénoïdale en cas d'ostéotomie sphénoïdale supra-tuberculaire, lequel instrument comprend une tige ayant une extrémité de percussion et une extrémité de fixation qui présente, pour la fixation de la lame, un mécanisme de serrage qui comporte un organe d'actionnement que l'on peut faire tourner au moyen d'un outil, caractérise en ce que l'organe d'actionnement (31) est disposé à proximité de l'extrémité de percussion (3), à l'extrémité d'une barre (30) qui est guidée le long de la tige (1).

2. Instrument selon la revendication 1, caractérisé en ce que l'organe d'actionnement (31) est disposé en retrait dans une forure (35) de guidage de l'outil, formée dans l'extrémité de percussion.

3. Instrument selon la revendication 1 ou 2, caractérisé en ce que le mécanisme de serrage comprend un pilon (5) raccordé rigidement à la tige (1) et une pince (15, 17) qui peut être serrée contre le pilon (5), en enserrant une partie (21) de la lame (23), au moyen d'une vis (29) reliée à l'organe d'actionnement (31).

4. Instrument selon la revendication 3, caractérisé en ce que la pince (15, 17) comprend deux parois latérales (15) situées sur les côtés du pilon et des saillies de serrage (17) disposées aux extrémités de ces parois et dirigées l'une vers l'autre, saillies qui délimitent, avec la surface frontale (7) du pilon (5), une chambre (19) ouverte perpendiculairement à la direction de la tige, pour recevoir une plaque de tête (21) de la lame.

Fig. 1

Fig. 2

Fig. 3